# EUROPEAN PATENT SPECIFICATION

(11) **EP 1 242 138 B1**
(45) Date of publication and mention of the grant of the patent: **02.05.2007**
(21) Application number: 00986468.7
(22) Date of filing: 14.12.2000
(51) Int. Cl.: A61M 25/01

(54) **COMPOSITE GUIDEWIRE INCLUDING AN AXIALLY MOVABLE CORE MEMBER**
KOMPOSITFÜHRUNGSDRACHT MIT EINEM AXIAL BEWEGLICHEN KERNGLIED
CABLE GUIDE COMPOSITE COMPRENANT UN ELEMENT NOYAU POUVANT ETRE DEPLACE DE MANIERE AXIALE

(30) Priority: 21.12.1999 US 468976
(43) Date of publication of application: 25.09.2002
(73) Proprietor: ADVANCED CARDIOVASCULAR SYSTEMS, INC., Santa Clara, California 95052-8167 (US)
(72) Inventor: BIAGTAN, Emmanuel C., Temecula, CA 92591 (US); ANDERSON, David, M., Temecula, CA 92592 (US); BAJAJ, Shruti, Temecula, CA 92591 (US); CORNISH, Wayne, Fallbrook, CA 92028 (US); GOISIENGFIAO, Brandon, Temecula, CA 92592 (US); MAHIEU, Ed, Hemet, CA 92544 (US); RICHARDSON, Mark, Escondido, CA 92026 (US)
(74) Representative: McLeish, Nicholas Alistair Maxwell
(86) International application number: PCT/US2000/034168
(87) International publication number: WO 2001/045786

(56) References cited:
- EP-A- 0 468 645
- EP-A- 0 820 782
- US-A- 4 657 024

## Description

### BACKGROUND

This invention relates to the field of guidewires for advancing intraluminal devices such as stent delivery catheters, balloon dilatation catheters, atherectomy catheters and the like within a patient's body, specifically, within a patient's vasculature.

In a typical percutaneous procedure in a patient's coronary system, a guiding catheter having a preformed distal tip is percutaneously introduced into a patient's peripheral artery, e.g. femoral, radial or brachial artery, by means of a conventional Seldinger technique and advanced therein until the distal tip of the guiding catheter is seated in the ostium of a desired coronary artery. There are two basic techniques for advancing a guidewire into the desired location within the patient's coronary anatomy, the first is a preload technique which is used primarily for over-the-wire (OTW) devices and the bare wire technique which is used primarily for rail type systems. With the preload technique, a guidewire is positioned within an inner lumen of an OTW device such as a dilatation catheter or stent delivery catheter with the distal tip of the guidewire just proximal to the distal tip of the catheter and then both are advanced through the guiding catheter to the distal end thereof. The guidewire is first advanced out of the distal end of the guiding catheter into the patient's coronary vasculature until the distal end of the guidewire crosses the arterial location where the interventional procedure is to be performed, e.g. a lesion to be dilated or a dilated region where a stent is to be deployed.

The catheter, which is slidably mounted onto the guidewire, is advanced out of the guiding catheter into the patient's coronary anatomy over the previously introduced guidewire until the operative portion of the intravascular device, e.g. the balloon of a dilatation or a stent delivery catheter, is properly positioned across the arterial location. Once the catheter is in position with the operative means located within the desired arterial location, the interventional procedure is performed. The catheter can then be removed from the patient over the guidewire. Usually, the guidewire is left in place for a period of time after the procedure is completed to ensure reaccess to the arterial location if it is necessary. For example, in the event of arterial blockage due to dissected lining collapse, a rapid exchange type perfusion balloon catheter such as described and claimed in U.S. Patent 5,516,336 (McInnes et al), can be advanced over the in-place guidewire so that the balloon can be inflated to open up the arterial passageway and allow blood to perfuse through the distal section of the catheter to a distal location until the dissection is reattached to the arterial wall by natural healing.

With the bare wire technique, the guidewire is first advanced by itself through the guiding catheter until the distal tip of the guidewire extends beyond the arterial location where the procedure is to be performed. Then a rail type catheter, such as described in U.S. Patent No. 5,061,395 (Yock) and the previously discussed McInnes et al., is mounted onto the proximal portion of the guidewire which extends out of the proximal end of the guiding catheter which is outside of the patient. The catheter is advanced over the guidewire, while the position of the guidewire is fixed, until the operative means on the rail type catheter is disposed within the arterial location where the procedure is to be performed. After the procedure the intravascular device may be withdrawn from the patient over the guidewire or the guidewire advanced further within the coronary anatomy for an additional procedure.

Conventional guidewires for angioplasty, stent delivery, atherectomy and other vascular procedures usually comprise metallic elongated core member with one or more tapered sections near the distal end thereof and a flexible body such as a metallic helical coil or a tubular body of polymeric material disposed about the distal portion of the core member. A shapable member, which may be the distal extremity of the core member or a separate shaping ribbon which is secured to the distal extremity of the core member, extends through the flexible body and is secured to the distal end of the flexible body by soldering, brazing or welding which forms a rounded distal tip. Torquing means are provided on the proximal end of the core member to rotate, and thereby steer, the guidewire while it is being advanced through a patient's vascular system.

Further details of guidewires, and devices associated therewith for various interventional procedures can be found in U.S. Patent 4,748,986 (Morrison et al.); U.S. Patent 4,538,622 (Samson et al.): U.S. Patent 5,135,503 (Abrams); U.S. Patent 5,341,818 (Abrams et al.); U.S. Patent 5,345,945 (Hodgson, et al.) and U.S. Patent 5,636,641 (Fariabi). For example, EP-A2-0,820,782 describes a composite guidewire for use in a catheter. It is used in medical procedures that involve accessing specifically targeted inner body areas without major surgery and is said to be especially useful for accessing peripheral soft tissue targets. The guidewire comprises a multi-section guidewire assembly having superelastic alloy ribbon braided reinforcements along at least a portion of the core. The guidewire core may be of a stainless steel or a high elasticity metal alloy, preferably a Ni-Ti alloy. A variation of the guidewire also includes a braid on the exterior of the core wire with an exterior polymeric coating to the most distal portion of the assembly and is assembled using adhesives such as epoxies. Similarly, EP-A1-0,468,645 describes a movable core guidewire for use in guiding a catheter to an internal body location which includes an elongate, flexible helical coil having a lumen extending longitudinally therethrough for receiving a movable core wire. The movable core wire has a flexible polymeric element extending from the distal end which facilitates smooth movement of the moveable core wire within the lumen of the helical coil.

Conventional metallic guidewires using tapered distal core sections as discussed above can be difficult to use with sensitive imaging systems such as MRI and the like because the metal content of the guidewire can create imaging artifacts that obscure the image produced. What has been needed is a guidewire that is compatible for use with sensitive imaging systems and methods such as MRI and the like.

### SUMMARY

The invention is directed to an intracorporeal guiding device which can be in the form of a guidwire. According to a first aspect of the present invention there is provided an intracorporeal guiding device comprising an elongate member having a proximal section and a distal section made at least partially of a fiber composite matrix, the elongate member having a core lumen and at least one segment with increasing flexibility in a distal direction, and an elongate core member disposed within the core lumen of the elongate member, the core member being comprised of a fiber composite matrix. The fiber composite matrix can be configured to have little or no metal content so as to avoid creating imaging artifacts with sensitive imaging systems such as MRI and the like. In one embodiment, a flexible body is disposed about the distal section of the elongate member. The flexible body can have a variety of configurations, including a helical coil and a polymer layer. In a particular embodiment, the flexible body which consists of a polymer layer can be doped with a radiopaque material in order to improve visualization of the device under fluoroscopic imaging and the like.

The elongate core can be fixed or secured within the core lumen, or it may be moveable in an axial direction. Movement of the elongate core within the core lumen of the device may be used to adjust the flexibility of the distal section.

In another embodiment, a shapeable segment can be secured to the distal end of the elongate member with the flexible body disposed at least partially about the shapeable segment. In some embodiments, the shapeable segment is comprised of metal which can be flattened to provide improved shapeability in a specified orientation.

The invention is also directed to a method of making an elongate intracorporeal guiding device. The method includes disposing at least one layer of thin fiber about a mandrel. This can be done by winding, stranding, braiding or any other suitable method. A binding agent is then applied to the fiber material. If necessary, the binding agent can then be cured. Alternatively, a binding agent may be present on a thin fiber prior to disposing the thin fiber on the mandrel.

The elongate intracorporeal guiding device may be advanced within a patient's body by providing the elongate intracorporeal guiding device with a distal section configured so as not to create imaging artifacts when used with MRI imaging. The elongate intracorporeal guiding device is then inserted into the patient's body and advanced within the patient's body under MRI imaging to a desired site. A distal section configured to not create imaging artifacts with MRI imaging, or other sensitive imaging methods, can be a distal section constructed essentially of non-metallic fiber composite matrix optionally including polymer materials having little or not metallic content.

### BRIEF DESCRIPTION OF THE DRAWINGS

FIG. 1 is an elevational view in partial section of an intracorporeal guiding device having features of the invention.
FIG. 2 is a transverse cross sectional view of the intracorporeal guiding device of FIG. 1 taken along lines 2-2 in FIG. 1.
FIG. 3 is a transverse cross sectional view of the intracorporeal guiding device of FIG. 1 taken along lines 3-3 in FIG. 1.
FIG. 4 is a transverse cross sectional view of the intracorporeal guiding device of FIG. 1 taken along lines 4-4 in FtG. 1.
FIG. 5 is an elevational view in partial section of a part of a distal section of an intracorporeal guiding device having features of the invention.
FIG. 6. is a transverse cross sectional view of the intracorporeal guiding device of FIG. 5 taken along lines 6-6 in FIG. 5.
FIG. 7 is an elevational view in partial section of an intracorporeal guiding device having features of the invention.
FIG. 8 is a transverse cross sectional view of the intracorporeal guiding device of FIG. 7 taken along lines 8-8 in FIG. 7.
FIG. 9 is a schematic view of thin fibers being braided onto a mandrel.
FIG. 10 is a schematic view of a thin fiber being wound onto a mandrel.
FIG. 11 is a schematic view of thin fibers being stranded onto a mandrel.

### DETAILED DESCRIPTION

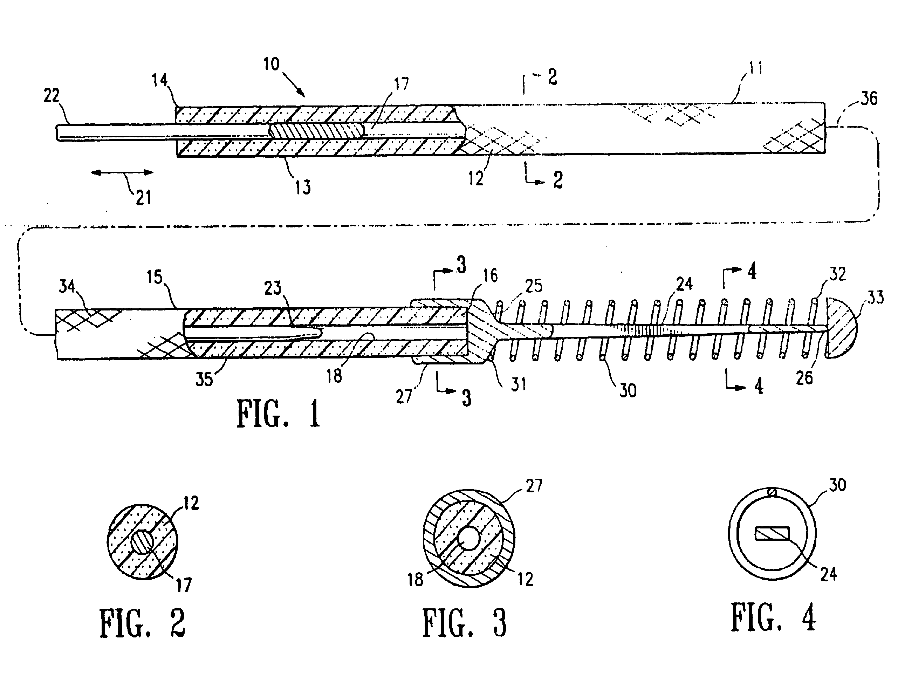

FIG. 1 shows an intracorporeal guiding device 10 having features of the invention. An elongate member 11 made entirely of fiber composite matrix 12 has a proximal section 13, a proximal end 14, a distal section 15 and a distal end 16. Optionally, the elongate member 11 can be made partially out of fiber composite matrix 12. A core member 17 is disposed within a core lumen 18 of the elongate member 11 and is axially moveable within the core lumen 18 as indicated by arrow 21. The core member 17 has a proximal end 22 and a distal end 23 and may also be secured within the core lumen 18 either by frictional force, an epoxy or other adhesive, or by any other suitable means. An optional shapeable segment 24 having a proximal end 25 and a distal end 26 has an end cap 27 disposed at the proximal end 25 of the shapeable segment 24. The end cap 27 is disposed over and - secured to the distal end 16 of the elongate member 11. The end cap 27 may be secured to the distal end 16 of the elongate member 11 by a friction fit, adhesive such as an epoxy, or any other suitable method. A flexible body in the form of a helical coil 30 having a proximal end 31 and a distal end 32 is disposed about the shapeable segment 24. The distal end 32 of the helical coil 30 is secured to the distal end 26 of the shapeable segment 24 with a body of solder 33 or the like. The proximal end 31 of the helical coil 30 can be similarly secured to the proximal end 25 of the shapeable segment 24.

The fiber composite matrix 12 of the elongate member 11 may be formed in a variety of configurations and from a variety of materials. In the embodiment of the intracorporeal guiding device 10 of FIG. 1, the fiber composite matrix 12 can be formed from a plurality of non-metallic thin fibers 34 made of carbon fiber braided over a mandrel (not shown) in one or more layers. A cured or hardened binding agent 35 such as an epoxy resin, polyester resin or other suitable material is disposed about the thin fibers 34 to form the fiber composite matrix 12. The binding agent 35 can optionally be doped with a radiopaque material in order to provide radiopacity to the elongate member 11. Materials such as gold, platinum, platinum-iridium, tungsten, barium compounds or bismuth compounds may be used for doping the binding agent 35. In addition, one or more radiopaque thin fibers 34 can be made of the same or similar radiopaque materials discussed above with regard to radiopaque dopants for the binding agent and may be used when forming the fiber composite matrix 12 in order to provide radiopacity to the device 10. In addition, a conductor, insulated or uninsulated, or other type of conduit capable of carrying an electric, light, or other type of signal can be substituted or molded into the thin fibers 34. This can be done in order to carry a signal conveying information such as temperature or pressure from the distal end 16 to the proximal end 14 of the elongate member 11. If a fiber optic is used, light or a light signal can be transmitted from the distal end 16 to the proximal end 14 of the elongate member 11, or from the proximal end 14 to the distal end 16. During formation of the elongate member 11, the aforementioned mandrel is removed after the binding agent is cured.

During the formation process for the elongate member 11, the core member 17 could serve as a forming mandrel such as that discussed above, or a separate mandrel could be used for forming the fiber composite matrix 12 and then be removed. A core member 17 could then be inserted into the core lumen 18 once the forming mandrel is removed. The thin fiber 34 could also be wound or stranded about the forming mandrel prior to curing of the binding agent. Any appropriate number of layers of thin fiber 34 may be braided, stranded or wound about a forming mandrel in order to achieve a desired thickness of the fiber composite matrix 12. For an intracorporeal guiding member 10 having a elongate member 11 with a proximal section 13 having an outer diameter of about 0.20mm to about 1.02mm (about 0.008 to about 0.040 inches), approximately 1 to about 10 layers of thin fiber 34 may be used, specifically, about 2 to about 6 layers of thin fiber. The thin fibers 34 can have a transverse dimension of about 0.013mm to about 0.051mm (about 0.0005 to about 0.002 inches), specifically about 0.025mm to about 0.038mm (about 0.001 to about 0.0015 inches) and can be made of carbon fiber. Other materials that can be used for the thin fibers 34 may include Nylon; Kevlar, fiberglass and the like.

The flexibility of the elongate member 11 can be controlled to some degree by varying the manner in which the one or more thin fibers are configured within the fiber composite matrix with respect to axial position along the elongate member 11. Specifically, the angle the thin fiber 34 makes with a line parallel to a longitudinal axis 36 of the elongate member 12 adjacent the thin fiber 34 can affect the longitudinal flexibility of the elongate member and hence the intracorporeal guiding device 10. In addition, the distal section 15 of the elongate member 11 can be tapered to a reduced outer transverse dimension distally in one or more segments in order to increase the flexibility of such a segment. The tapering of a segment can be achieved by grinding a segment of substantially constant outer diameter after formation of the elongate member 11. Alternatively, the tapering of a segment could be achieved by varying the number of layers or configuration of the thin fiber or fibers 34 in the formation process of the elongate member 11. Also, the diameter of the core lumen 18 within the elongate member 11 could be increased distally in a segment of the distal section 15 of the elongate member 11 in order to increase the flexibility of the segment.

The core member 17 may be made of a fiber composite material similar to that of the elongate member 11 with a binding agent for such a fiber composite material being doped with a non-metallic radiopaque material in order to provide radiopacity to the core member 17 and avoid introduction of metallic content which might interfere with sensitive imaging methods as discussed above. The core member 17 can have an outer transverse dimension of about 0.025mm to about 0.381mm (about 0.001 to about 0.015 inches), specifically about 0.051mm to about 0.127mm (about 0.002 to about 0.005 inches). The core member 17 can also be ground to have one or more tapered segments, specifically, tapered segment tapering distally to a reduced transverse dimension in order to provide greater flexibility in the distal section 15 of the elongate member 11.

Generally, the shapeable segment 24 can have a configuration similar to shapeable segments of guiding devices known in the art. Regarding the embodiment of the guiding device 10 shown in FIG. 1, the shapeable segment 24 is formed of stainless steel which has optionally been flattened. Specifically, the shapeable segment 24 has been flattened to a progressively greater degree in a distal direction. Thus, a thickness of the flattened portion at the proximal end 25 of the shapeable segment 24 is thicker than the thickness of the flattened portion of the shapeable segment 24 at the distal end 26 of the shapeable segment 24. The length of the flexible segment 24 can be from about 2 to about 30 cm, specifically, about 3 to about 10 cm. The thickness of the shapeable segment 24 at the flattened distal end can be from about 0.013mm to about 0.152mm (about 0.0005 to about 0.006 inches), specifically, about 0.025mm to about 0.051mm (about 0.001 to about 0.002 inches). Other materials suitable for the shapeable segment 24 include MP35N, L605 or other high strength materials.

The helical coil 30 can be made from a variety of suitable materials including stainless steel, platinum, platinum iridium, gold or the like. The helical coil 30 could also be made from a fiber composite matrix or other non-metal material in order to enable the intracorporeal guiding device 10 to have a distal section or an overall composition with a zero or minimum amount of metallic composition. As mentioned above, for certain applications and uses, minimizing the metallic content of the intracorporeal guiding device 10 improves compatibility with sensitive imaging devices such as MRI. The material of the helical coil 30 can have a transverse dimension of about 0.025mm to about 0.127mm (about 0.001 to about 0.005 inches), specifically, about 0.051mm to about 0.076mm (about 0.002 to about 0.003 inches).

The nominal outer transverse dimension of the proximal section 13 of the elongate member 11 can be from about 0.127mm to about 0.889mm (about 0.005 to about 0.035 inches), specifically about 0.254mm to about 0.508mm (about 0.01 to about 0.02 inches) and more specifically about 0.305mm to about 0.406mm (about 0.012 to about 0.016 inches). The overall length of the intracorporeal guiding device 10 can be from about 100 to about 300 cm, specifically about 150 to about 200 cm.

FIG. 2 is a transverse cross sectional view of the intracorporeal guiding device 10 of FIG. 1 taken along lines 2-2 in FIG. 1. The fiber composite matrix 12 is substantially concentrically disposed about the core member 17 as discussed above. In FIG. 3, the end cap 27 is disposed about the fiber composite matrix 12 which is substantially concentrically disposed about the core lumen 18. In FIG. 4, the helical coil 30 is disposed about the shapeable segment 24.

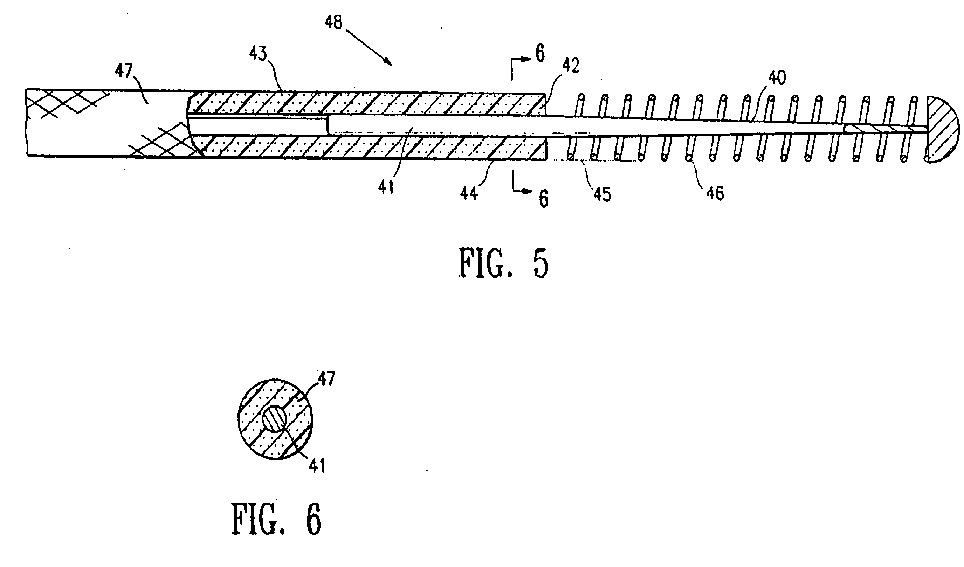

FIGS. 5 and 6 depict an alternative embodiment of a shapeable segment 40 wherein the end cap 27 of the shapeable segment 24 of FIG. 1 has been replaced with a handle portion 41 which is disposed within and secured to the distal end 42 of the elongate member 43. A configuration such as that shown in FIG. 5 allows for a smooth continuous transition from an outer surface 44 of the elongate member 43 to an outer surface 45 of the helical coil 46. A fiber composite matrix 47 is substantially concentrically disposed about the handle portion 41 of the shapeable segment 40. Components of the embodiment of the intracorporeal guiding device 48 shown in FIGS. 5 and 6 could have similar relationships, dimensions and materials to similar components of the embodiment of the intracorporeal guiding device 10 shown in FIGS. 1-4.

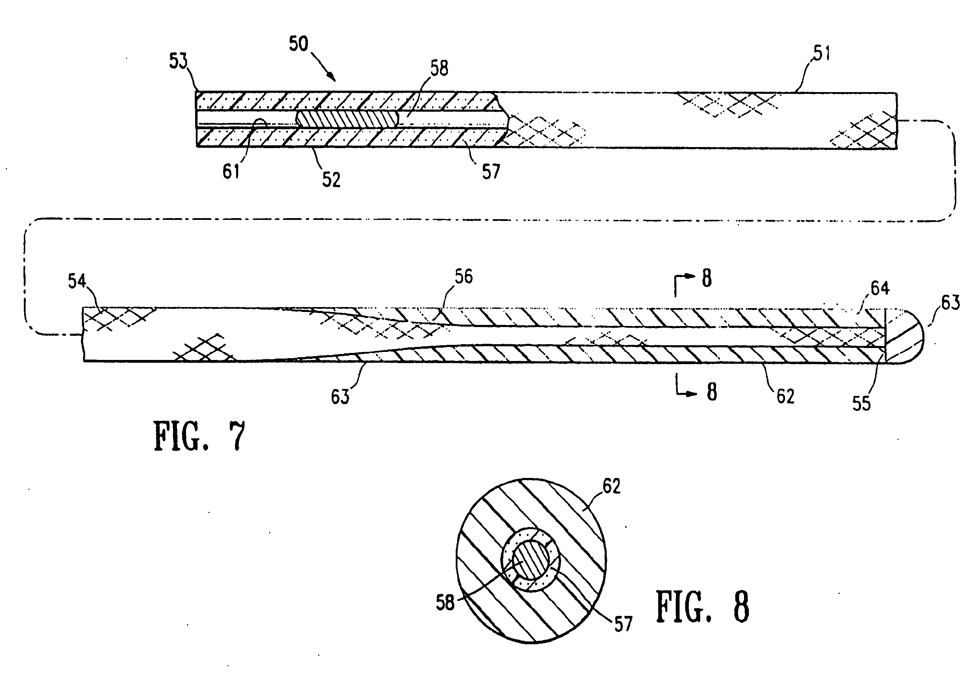

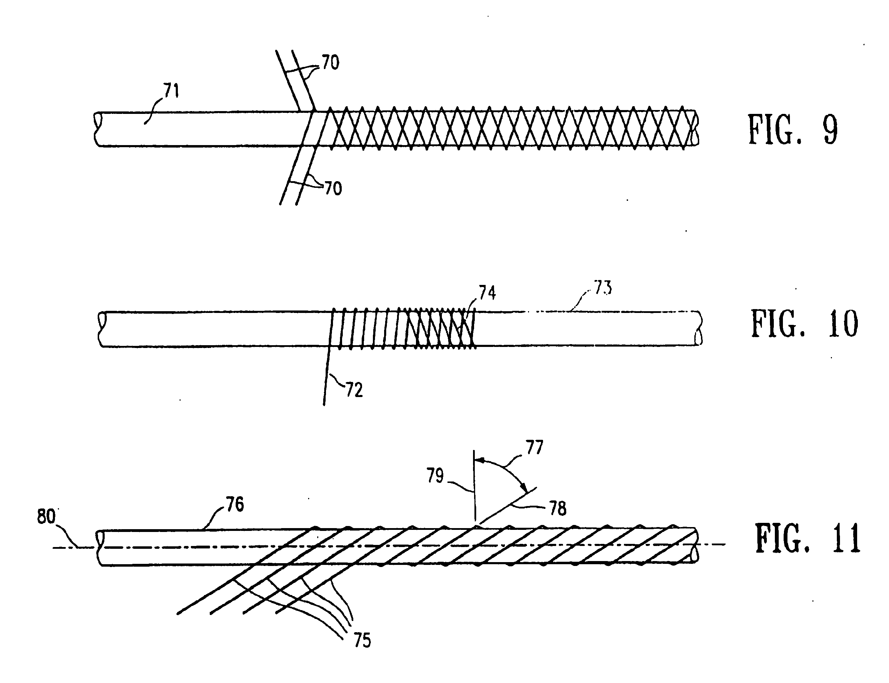

FIGS. 7 and 8 show another embodiment of an intracorporeal guiding device 50 having features of the invention. An elongate member 51 has a proximal section 52, a proximal end 53, a distal section 54 and a distal end 55. The distal section 54 has a tapered segment 56 which tapers distally to a reduced transverse dimension in order to increase the flexibility of the distal section 54. The elongate member 51 is formed of a fiber composite matrix 57 such as that described above with regard to other embodiments of the invention. A core member 58 is secured within a core lumen 61 of the elongate member 51. The core member 58 can be made of a non-metallic fiber composite matrix or other essentially non-metallic material in order to avoid interference with sensitive imaging systems such as MRI and the like. The tapered segment 56 of the distal section 54 of the elongate member 51 tapers in a curved configuration which can provide a smooth transition in flexibility. A flexible body in the form of a polymer layer 62 is substantially concentrically disposed about the distal section 54 of the elongate member 51. A rounded polymer cap 63 is secured to the distal end 55 of the elongate member 51 to facilitate securement of the polymer layer 62 to the elongate member 51 and to provide a rounded non-traumatic tip for the intracorporeal guiding device 50. The rounded polymer cap 63 can be a separate element as shown in FIG. 7, or it may be a continuation and integral portion of polymer layer 62. The polymer layer 62 has a proxial end 63 and distal end 64.

The polymer layer 62 can be made from a diverse range of materials, including polyurethane, polyethylene, Nylon, silicone, or any other suitable polymer. The polymer layer 62 can optionally be doped with a radiopaque material in order to facilitate imaging of the guiding device 50 under flouroscopy. The polymer layer 62 can be applied by coextrusion, heat shrink, bonding with a suitable adhesive or any other appropriate method. The polymer layer 62 can be formed on the distal section 54 of the elongate member 51 or may be extruded independently and later secured to the distal section 54. The length and outer dimensions of the polymer layer 62 can be similar to those of the helical coil 30 discussed above. FIG. 8 is a transverse cross sectional view of the intracorporeal guiding device 50 of FIG. 7 taken along lines 8-8 in FIG. 7. The polymer layer 62 is shown substantially concentrically disposed about the fiber composite matrix 57 which is substantially concentrically disposed about the core member 58. Components of the embodiment of the intracorporeal guiding device 50 shown in FIGS. 7 and 8 could have similar relationships, dimensions and materials to similar components of the embodiment of the intracorporeal guiding device 10 shown in FIGS. 1-6.

FIG. 9 illustrates four thin fibers 70 being braided onto a mandrel. FIG. 10 illustrates a single thin fiber 72 being wound onto a mandrel 73. A double layer section 74 is shown where the thin fiber 72 been wound back onto itself in order to form two layers. FIG. 11 shows four thin fibers 75 being stranded onto a mandrel 76. Also shown is the pitch angle 77 that a line 78 extending from one of the thin fibers 75 makes with a line 79 orthogonal to a longitudinal axis 80 of the mandrel 76. The pitch angle 77 of stranded, braided or wound thin fiber 75 can vary significantly. The pitch angle 77 can be just over zero degrees for a single thin fiber 75 being wound close spaced so that adjacent windings are touching each other. The pitch angle 77 can be up to 90 degrees for multiple stranded thin fibers 75 which extend essentially parallel to the longitudinal axis 80 of the mandrel 76. In one embodiment, the pitch angle 77 can be from about 20 to about 70 degrees, specifically, about 30 to about 60 degrees, and more specifically about 40 to about 50 degrees. Such variations in pitch angle 77 can be used to control the flexibility of the resulting elongate member for a fixed cross section of fiber composite material.

White particular forms of the invention have been illustrated and described, it will be apparent that various modifications can be made without departing from the scope of the invention. Accordingly, it is not intended that the invention be limited, except as by the appended claims.

## Claims

1. An intracorporeal guiding device comprising an elongate member (11) having a proximal section (13) and a distal section (15) made at least partially of a fiber composite matrix (12), the elongate member (11) having a core lumen (18) and at least one segment with increasing flexibility in a distal direction, and
an elongate core member (17) disposed within the core lumen (18) of the elongate member (11), **characterised in that** the core member (17) being comprised of a fiber composite matrix.

2. The intracorporeal guiding device of claim 1 wherein the fiber composite matrix of the elongate member (11) is comprised of a material selected from the group consisting of carbon fiber, fiberglass, Nylon and Kevlar.

3. The intracorporeal guiding device of claim 1 further comprising a flexible body (30,62) disposed about the distal section (15) of the elongate member (11).

4. The elongate intracorporeal guiding device of claim 3 wherein the flexible body (30,62) is comprised of a helical coil (30).

5. The elongate intracorporeal guiding device of claim 3 wherein the flexible body (30,62) is comprised of a polymer layer (62).

6. The elongate intracorporeal guiding device of claim 5 wherein the polymer layer (62) is doped with a radiopaque material.

7. The elongate intracorporeal guiding device of claim 4 wherein the helical coil (30) is made of a non-metal fiber composite matrix.

8. The elongate intracorporeal guiding device of claim 7 wherein the non-metal fiber composite matrix of the helical coil (30) is doped with a radiopaque material.

9. The elongate intracorporeal guiding device of claim 1 wherein the core member (17) is radiopaque.

10. The elongate intracorporeal guiding device of claim 1 wherein the core member (17) is axially movable within the core lumen (18) of the elongate member (11).

11. The elongate intracorporeal guiding device of claim 1 wherein the composite fiber matrix comprises at least one thin fiber in a stranded configuration.

12. The elongate intracorporeal guiding device of claim 1 wherein the composite fiber matrix comprises at least one thin fiber in a wound configuration.

13. The elongate intracorporeal guiding device of claim 1 wherein the composite fiber matrix comprises at least one layer of thin fibers in a braided configuration.

14. The elongate intracorporeal guiding device of claim 1 further comprising a shapeable segment (24) having an end cap (27) configured to be disposed over and secured to a distal end (16) of the elongate member (11).

15. The elongate intracorporeal guiding device of claim 1 further comprising a shapeable segment (40) having a handle portion (41) configured to be disposed within and secured to a distal end (42) of the elongate member (43).

16. The elongate intracorporeal guiding device of claim 1 further comprising shapeable member (24,40) comprised of metal.

17. A method of making an elongate intracorporeal guiding device comprising:
disposing at least one layer of thin fiber material about a mandrel;
applying a binding agent to the fiber material to form an elongate member (11) having a core lumen (18); and
disposing an elongate core member (17) within the core lumen (18) of the elongate member (11), **characterised in that** the core member being comprised of a fiber composite matrix.

18. The method of claim 17 further comprising curing the binding agent after it has been applied to the fiber material.

19. The method of claim 17 further comprising removing the mandrel after the binding agent has been applied.

20. The method of claim 17 wherein the thin fiber material is applied by winding the material about the mandrel.

21. The method of claim 17 wherein the thin fiber material is applied by stranding the material about the mandrel.

22. the method of claim 17 wherein the thin fiber material is applied by braiding the material about the mandrel.

## Patentansprüche

1. Intrakorporale Führungsvorrichtung, umfassend ein längliches Element (11) mit einem proximalen Abschnitt (13) und einem distalen Abschnitt (15), das zumindest teilweise aus einer Faserverbundmatrix (12) besteht, wobei das längliche Element (11) ein Innenlumen (18) und wenigstens einen Abschnitt mit zunehmender Flexibilität in distaler Richtung besitzt, und ein längliches Innenelement (17), das innerhalb des Innenlumens (18) des länglichen Elementes (11) angeordnet ist, **dadurch gekennzeichnet, dass** das Innenelement (17) aus einer Faserverbundmatrix besteht.

2. Intrakorporale Führungsvorrichtung nach Anspruch 1, wobei die Faserverbundmatrix des länglichen Elementes (11) aus einem Material besteht, das ausgewählt ist aus der Gruppe bestehend aus Kohlenstofffaser, Fiberglas, Nylon und Aramit-Faser.

3. Intrakorporale Führungsvorrichtung nach Anspruch 1, weiter umfassend einen flexiblen Körper (30, 62), der sich an dem distalen Abschnitt (15) des länglichen Elementes (11) befindet.

4. Längliche intrakorporale Führungsvorrichtung nach Anspruch 3, wobei der flexible Körper (30, 62) aus einer spiralförmigen Wicklung (30) besteht.

5. Längliche intrakorporale Führungsvorrichtung nach Anspruch 3, wobei der flexible Körper (30, 62) aus einer Polymerschicht (62) besteht.

6. Längliche intrakorporale Führungsvorrichtung nach Anspruch 5, wobei die Polymerschicht (62) mit einem Kontrastmittel getränkt ist.

7. Längliche intrakorporale Führungsvorrichtung nach Anspruch 4, wobei die spiralförmige Wicklung (30) aus einer nicht metallischen Faserverbundmatrix besteht.

8. Längliche intrakorporale Führungsvorrichtung nach Anspruch 7, wobei die nicht metallische Faserverbundmatrix der spiralförmigen Wicklung (30) mit einem Kontrastmittel getränkt ist.

9. Längliche intrakorporale Führungsvorrichtung nach Anspruch 1, wobei das Innenelement (17) strahlenundurchlässig ist.

10. Längliche intrakorporale Führungsvorrichtung nach Anspruch 1, wobei das Innenelement (17) innerhalb des Innenlumens (18) des länglichen Elementes (11) axial bewegbar ist.

11. Längliche intrakorporale Führungsvorrichtung nach Anspruch 1, wobei die Faserverbundmatrix wenigstens eine dünne Faser in verdrillter Ausgestaltung umfasst.

12. Längliche intrakorporale Führungsvorrichtung nach Anspruch 1, wobei die Faserverbundmatrix wenigstens eine dünne Faser in einer gewundenen Ausgestaltung umfasst.

13. Längliche intrakorporale Führungsvorrichtung nach Anspruch 1, wobei die Faserverbundmatrix wenigstens eine Schicht von dünnen Fasern in einer umsponnenen Ausgestaltung umfasst.

14. Längliche intrakorporale Führungsvorrichtung nach Anspruch 1, weiter umfassend einen verformbaren Abschnitt (24) mit einer Endhaube (27), die so ausgebildet ist, dass sie über ein distales Ende (16) des länglichen Elementes (11) angebracht und daran gesichert werden kann.

15. Längliche intrakorporale Führungsvorrichtung nach Anspruch 1, weiter umfassend einen verformbaren Abschnitt (40) mit einem Griffelement (41), das so ausgebildet ist, dass es innerhalb eines distalen Endes (42) des länglichen Elementes (43) angeordnet und daran gesichert werden kann.

16. Längliche intrakorporale Führungsvorrichtung nach Anspruch 1, weiter umfassend ein verformbares Element (24, 40), das aus Metall besteht.

17. Verfahren zur Herstellung einer länglichen intrakorporalen Führungsvorrichtung, umfassend:
Anbringen von wenigstens einer Schicht von einem Material aus dünnen Fasern um einen Schaft;
Anwenden eines Bindungsmittels an das Fasermaterial zur Bildung eines länglichen Elementes (11) mit einem Innenlumen (18) und
Anbringen eines länglichen Innenelementes (17) innerhalb des Innenlumens (18) des länglichen Elementes (11), **dadurch gekennzeichnet, dass** das Innenelement aus einer Faserverbundmatrix besteht.

18. Verfahren nach Anspruch 17, weiter umfassend das Aushärten des Bindungsmittels, nachdem es auf das Fasermaterial angewendet wurde.

19. Verfahren nach Anspruch 17, weiter umfassend das Entfernen des Schaftes, nachdem das Bindungsmittel angewendet wurde.

20. Verfahren nach Anspruch 17, wobei das Material aus dünnen Fasern angewendet wird, indem das Material um den Schaft gewunden wird.

21. Verfahren nach Anspruch 17, wobei das Material aus dünnen Fasern angewendet wird, indem das Material um den Schaft verdrillt wird.

22. Verfahren nach Anspruch 17, wobei das Material aus dünnen Fasern angewendet wird, indem das Material um den Schaft umsponnen wird.

## Revendications

1. Dispositif de guidage intracorporel comprenant un élément allongé (11) ayant une section proximale (13) et une section distale (15) composé au moins partiellement d'une matrice composite fibreuse (12), l'élément allongé (11) ayant un lumen central (18) et au moins un segment avec une flexibilité croissante dans la direction distale, et
un élément central allongé (17) disposé à l'intérieur du lumen central (18) de l'élément allongé (11), **caractérisé en ce que** l'élément central (17) est constitué par une matrice composite fibreuse.

2. Dispositif de guidage intracorporel selon la revendication 1, dans lequel la matrice composite fibreuse de l'élément allongé (11) est composée d'un matériau sélectionné dans le groupe constitué par la fibre de carbone, la fibre de verre, le Nylon et le Kevlar.

3. Dispositif de guidage intracorporel selon la revendication 1, comprenant en outre un corps flexible (30, 62) disposé autour de la section distale (15) de l'élément allongé (11).

4. Dispositif de guidage intracorporel allongé selon la revendication 3, dans lequel le corps flexible (30, 62) est constitué par une bobine hélicoïdale (30).

5. Dispositif de guidage intracorporel allongé selon la revendication 3, dans lequel le corps flexible (30, 62) est constitué par une couche polymère (62).

6. Dispositif de guidage intracorporel allongé selon la revendication 5, dans lequel la couche polymère (62) est dopée avec un matériau radio-opaque.

7. Dispositif de guidage intracorporel allongé selon la revendication 4, dans lequel la bobine hélicoïdale (30) est constituée par une matrice composite fibreuse non métallique.

8. Dispositif de guidage intracorporel allongé selon la revendication 7, dans lequel la matrice composite fibreuse non métallique de la bobine hélicoïdale (30) est dopée avec un matériau radio-opaque.

9. Dispositif de guidage intracorporel allongé selon la revendication 1, dans lequel l'élément central (17) est radio-opaque.

10. Dispositif de guidage intracorporel allongé selon la revendication 1, dans lequel l'élément central (17) peut être déplacé axialement dans le lumen central (18) de l'élément allongé (11).

11. Dispositif de guidage intracorporel allongé selon la revendication 1, dans lequel la matrice fibreuse composite comprend au moins une fibre mince dans une configuration toronnée.

12. Dispositif de guidage intracorporel allongé selon la revendication 1, dans lequel la matrice fibreuse composite comprend au moins une fibre mince dans une configuration enroulée.

13. Dispositif de guidage intracorporel allongé selon la revendication 1, dans lequel la matrice fibreuse composite comprend au moins une couche de fibres minces dans une configuration tressée.

14. Dispositif de guidage intracorporel allongé selon la revendication 1, comprenant en outre un segment pouvant être façonné (24) ayant un bouchon d'extrémité (27) configuré pour être disposé sur et fixé à une extrémité distale (16) de l'élément allongé (11).

15. Dispositif de guidage intracorporel allongé selon la revendication 1, comprenant en outre un segment pouvant être façonné (40) ayant une partie de préhension (41) configurée pour être disposée dans et fixée à une extrémité distale (42) de l'élément allongé (43).

16. Dispositif de guidage intracorporel allongé selon la revendication 1, comprenant en outre un élément pouvant être façonné (24, 40) composé de métal.

17. Procédé de fabrication d'un dispositif de guidage intracorporel allongé comprenant les étapes consistant à :
disposer au moins une couche de matériau fibreux mince autour d'un mandrin ;
appliquer un agent de liaison au matériau fibreux pour former un élément allongé (11) ayant un lumen central (18) ; et
disposer un élément central allongé (17) dans le lumen central (18) de l'élément allongé (11), **caractérisé en ce que** l'élément central est constitué par une matrice composite fibreuse.

18. Procédé selon la revendication 17, comprenant en outre le séchage de l'agent de liaison après son application au matériau fibreux.

19. Procédé selon la revendication 17, comprenant en outre le retrait du mandrin après l'application de l'agent de liaison.

20. Procédé selon la revendication 17, dans lequel le matériau fibreux mince est appliqué en enroulant le matériau autour du mandrin.

21. Procédé selon la revendication 17, dans lequel le matériau fibreux mince est appliqué en toronnant le matériau autour du mandrin.

22. Procédé selon la revendication 17, dans lequel le matériau fibreux mince est appliqué en tressant le matériau autour du mandrin.
